# EUROPEAN PATENT APPLICATION

(11) **EP 4 279 499 A1**
(43) Date of publication of application: **22.11.2023**
(21) Application number: 22739824.5
(22) Date of filing: 18.01.2022
(51) Int. Cl.: C07K 7/08, C07K 19/00, C07K 16/00, G01N 33/53, G01N 33/58, G01N 33/68

(54) **SWITCHING PEPTIDE AND IMMUNOASSAY USING SAME**

(30) Priority: 18.01.2021 KR 20210006850
(71) Applicant: Optolane Technologies Inc., Bundang-gu Seongnam-si, Gyeonggi-do 13494 (KR)
(72) Inventor: PYUN, Jae-Chul, Seoul 06521 (KR)
(74) Representative: Schiweck Weinzierl Koch Patentanwälte Partnerschaft mbB
(86) International application number: PCT/KR2022/000864
(87) International publication number: WO 2022/154632

(57) **Abstract**

Disclosed is a switching peptide that can be applied to an electrochemical immunoassay. The switching peptide includes: a peptide compound capable of binding reversibly to a binding antibody; and a chemical marker grafted to the peptide compound.

## Description

### [Technical Field]

The present disclosure relates to a switching peptide that may be used to analyze a target antigen substance using an immune response and an immunoassay method using the same.

### [Background Art]

Immunoassay has been used for bio-testing such as various immunodiagnostic tests or environmental monitoring in the field of biotechnology. The immunoassay has the advantage of being able to quantitatively analyze a specific target substance by using the specificity of an antigen-antibody reaction and having higher sensitivity than other analysis methods.

The immunoassay may be used for various diagnoses such as diagnosing cancer markers, infectious diseases, thyroid function, anemia, allergy, pregnancy, drug abuse, and gout. In order to use the immunoassay for diagnosis or analysis of various types of antigens, it is necessary to prepare antibodies having specific reactivity to each of the antigens.

However, such a method has limitations in detecting modernized diseases that have rapidly been diversified recently, or viruses having multiple mutants. In particular, in the case of the virus, it is often impossible to find an antibody having a specific binding property with the virus.

In addition, when the antigen-antibody reaction is used, an additional step of binding the antibody to a label may be required. If the binding property between the label and the antibody is poor, the accuracy or reliability of immunoassay analysis may be deteriorated.

Due to the aforementioned problems, there is also a problem of developing various types of labels capable of binding to various antibodies, respectively.

### [Disclosure]

### [Technical Problem]

One object of the present disclosure is to provide a switching peptide that may be applied in an electrochemical immunoassay method, comprising a peptide compound capable of selectively and reversibly binding to a Fab region of a binding antibody and a chemical label capable of being oxidized and reduced in a detection sample solution.

Another object of the present disclosure is to provide an immunoassay method using the switching peptide.

### [Technical Solution]

A switching peptide according to an embodiment of the present disclosure comprises: a peptide compound capable of reversibly binding to a binding antibody; and a chemical label bound to the peptide compound.

In one embodiment, the peptide compound may be selectively and reversibly bound to a fragment antigen-binding (Fab) region of the binding antibody.

In one embodiment, the peptide compound may have an amino acid sequence capable of specifically and reversibly binding to one or more of first to fourth framework regions (FR1, FR2, FR3, and FR4) of a light chain or heavy chain of the binding antibody.

In one embodiment, the peptide compound may comprise one or more selected from the group consisting of a first peptide compound having a first amino acid sequence having homology to an amino acid sequence of the second light chain variable framework region (VL-FR2); a second peptide compound having a second amino acid sequence having homology to an amino acid sequence of the third or fourth light chain variable framework region (VL-FR3, VL-FR4) ; a third peptide compound having a third amino acid sequence having homology to an amino acid sequence of the second heavy chain variable framework region (VH-FR2); and a fourth peptide compound having a fourth amino acid sequence having homology to an amino acid sequence of the third or fourth heavy chain variable framework region (VH-FR3, VH-FR4).

In one embodiment, the peptide compound may comprise 14 to 20 amino acids.

In one embodiment, the peptide compound may have a molecular weight of 1,650 to 2,500 Da.

In one embodiment, the chemical label may comprise a substance capable of being reversibly oxidized or reduced in a sample solution.

In one embodiment, the chemical label may comprise one or more selected from the group consisting of ferrocene, ferrocenemethanol, ferrocenedimethanol, α-methylferrocenemethanol, ferrocyanide ion, ferricyanide ion, hexaammineruthenium ion, hydroquinone, ascorbic acid, dopamine, ferrocene carboxylic acid, ferrocene dicarboxylic acid, and ferrocene aldehyde.

An immunoassay method according to an embodiment of the present disclosure includes: a first step of immobilizing a binding antibody to which a switching peptide is bound on a substrate or immobilizing the binding antibody on the substrate and then binding the switching peptide to the binding antibody; a second step of treating the binding antibody with a detection sample solution; and a third step of quantitatively analyzing a target antigen that specifically reacts with the binding antibody in the detection sample solution by performing electrochemical analysis on the detection sample solution after the treatment, wherein the switching peptide comprise a peptide compound having an amino acid sequence capable of selectively and reversibly binding to a fragment antigen-binding region (Fab) of the binding antibody, and a chemical label that is bound to the peptide compound and capable of being oxidized and reduced in the detection sample solution.

In one embodiment, when the target antigen is present in the detection sample solution in the second step, if the target antigen is bound to the binding antibody, the switching peptide may be quantitatively released from the binding antibody depending on an amount of the target antigen that has reacted with the binding antibody.

In one embodiment, the electrochemical analysis may be performed by one method selected from the group consisting of amperometry, chronoamperometry, voltammetry, cyclic voltammetry, differential potential voltammetry, chronopotentiometry, and polarography.

In one embodiment, a redox reaction may occur between a chemical label of the switching peptide released from the binding antibody in the third step and a working electrode for amperometry or voltammetry.

### [Advantageous Effects]

According to the switching peptide of the present disclosure and an immunoassay method using the same, by using a switching peptide selectively and reversibly bound to a Fab region of the binding antibody and having a chemical label, the target antigen may be quantitatively analyzed through an electrochemical analysis method that does not require an optical system, enabling miniaturization and low cost of a analysis device, and accordingly, it may be applied to a device for on-site diagnosis.

### [Description of Drawings]

FIG. 1 is a diagram for explaining a switching peptide according to an embodiment of the present disclosure.
FIG. 2 is a diagram for explaining a binding antibody.
FIG. 3A is a flowchart for explaining an immunoassay method according to an embodiment of the present disclosure, and FIGS. 3B and 3C are schematic diagrams for explaining one embodiment of the immunoassay method illustrated in FIG. 3A.
FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software.
FIG. 5 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.
FIG. 6A is a schematic diagram illustrating states before and after treatment of the antibody to which the four types of peptides of Example 1 were bound, respectively, with papain, which is a protease, and FIG. 6B is graphs illustrating a fluorescence intensity measured at a bottom (B) of the microplate and in a solution (S) after the papain treatment.
FIG. 7A is a schematic diagram for explaining an immunoassay method using fluorescently labeled peptide compounds of Example 1, and FIGS. 7B and 7C are graphs illustrating the fluorescence intensities in a solution and at a bottom of the microplate depending on a concentration of the antigen measured by the immunoassay method illustrated in FIG. 7A.
FIGS. 8A to 8C are results of analyzing switching peptides labeled with ferrocene.
FIGS. 9A and 9B are a graphs illustrating a change in current value depending on the concentration of an L1 switching peptide measured by a differential pulsed voltammetry (DPA) method for a sample solution containing the L1 switching peptide, and a graph illustrating a change in current value depending on the concentration of an H2 switching peptide measured by a differential pulsed voltammetry (DPA) method for a sample solution containing the H2 switching peptide, respectively.
FIG. 10 is results obtained by performing one-step immunoassay analysis on human hepatitis B antigen (hHBsAg) of the binding antibody bound to the L1 and H2 switching peptides according to the present disclosure.
FIGS. 11A and 11B are graphs illustrating analysis results depending on the concentration of antigen measured through an electrochemical immunoassay method using a first L1 switching peptide (L1 peptide compound having ferrocene bonded to a terminal) for a detection sample solution containing an HBsAg antigen, an optical immunoassay method using a second switching peptide (fluorescently labeled L1 peptide compound), a chemiluminescence-based immunoassay method using a conventional ELISA kit, and a chromogenic immunoassay method using a conventional ELISA kit.
FIGS. 12A and 12B are graphs obtained by performing Bland-Altman test and Passing-Bablok regression analysis for comparison with the conventional method of immunoassay using the peptide compounds of Example 2.

### [Best Mode for Invention]

Hereinafter, exemplary embodiments of the present disclosure will be described in detail with reference to the accompanying drawings. The present disclosure may be variously modified and have several exemplary embodiments. Therefore, specific exemplary embodiments of the present disclosure will be illustrated in the accompanying drawings and be described in detail in the specification. However, it is to be understood that the present disclosure is not limited to a specific disclosed form, but includes all modifications, equivalents, and substitutions without departing from the scope and sprit of the present disclosure. Similar reference numerals have been used for similar components in describing each drawing. In the accompanying drawings, dimensions of the structures have been enlarged as compared with the actual dimensions for clarity of the present disclosure.

Terms as used herein are used only in order to describe specific exemplary embodiments rather than limiting the present disclosure. Singular forms include plural forms unless the context clearly indicates otherwise. It should be understood that term "include" or "has" as used herein specify the presence of features, numerals, steps, operations, components described herein, or combinations thereof, but does not preclude the presence or addition of one or more other features, numerals, steps, operations, components, or combinations thereof.

Unless being defined otherwise, it is to be understood that all the terms as used herein including technical and scientific terms have the same meanings as those that are generally understood by one of ordinary skill in the art to which the present disclosure pertains. Terms generally used and defined by a dictionary should be interpreted as having the same meanings as meanings within a context of the related art and should not be interpreted as having ideal or excessively formal meanings unless being clearly defined otherwise in the present specification.

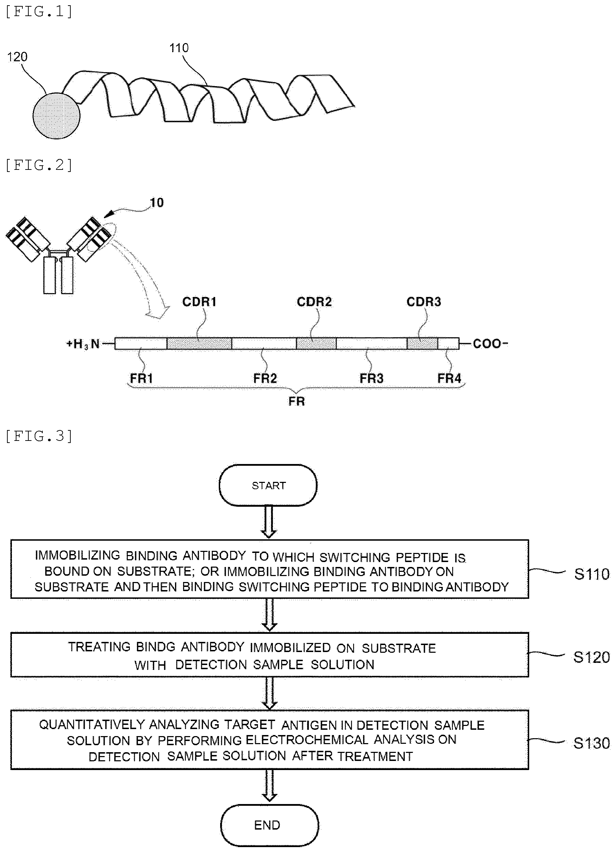

FIG. 1 is a diagram for explaining a switching peptide according to an embodiment of the present disclosure, and FIG. 2 is a diagram for explaining a binding antibody.

Referring to FIGS. 1 and 2, a switching peptide 100 according to an embodiment of the present disclosure may comprise a peptide compound 110 capable of reversibly binding to a binding antibody 10, and a chemical label 120 bound to the peptide compound 110. The switching peptide 100 may be reversibly bound to the binding antibody 10 that specifically bound to a target antigen, and thus may be quantitatively released from the binding antibody 10 when the target antigen is specifically bound to the binding antibody. As a result, the electrochemical characteristics of a test sample including the target antigen may be changed by a redox reaction mediated by the chemical label 120 of the switching peptide 100 released from the binding antibody 10.

The binding antibody 10 is a compound capable of inducing an immunological effector mechanism by binding to or reacting with a specific antigenic determinant such as an epitope of the target antigen. The binding antibody may be monospecific or polyspecific.

In one embodiment, the binding antibody 10 may comprise an antibody, an antibody derivative, or a fragment thereof. The binding antibody 10 may be an intact immunoglobulin molecule or, alternatively, may comprise components of intact antibodies such as Fab, Fab', F(ab')₂, Fc, F (v), N-glycan structure, paratope, etc., or at least some of the components.

In one embodiment, the binding antibody may be a human antibody, a non-human antibody, or a humanized non-human antibody.

The human antibody may comprise antibodies produced by humans, or synthetic antibodies having amino acid sequences synthesized using any technique. This definition of the human antibody specifically excludes humanized antibodies comprising non-human antigen-binding residues. The human antibody may be produced using a variety of techniques known in the art, including phage-display libraries. The non-human antibody may be an antibody obtained from sources of various species, for example, rodents, rabbits, cattle, sheep, pigs, dogs, non-human mammals, or birds. A "humanized" form of the non-human antibody may be a chimeric antibody that contains minimal sequence derived from non-human immunoglobulin. In one embodiment, the humanized antibody may be a human immunoglobulin (recipient antibody) in which residues from a hypervariable region of the recipient are replaced with the hypervariable region of the aforementioned non-human antibody (donor antibody) having a desired specificity, affinity and/or capacity. In some cases, framework residues of a human immunoglobulin may be replaced by corresponding non-human residues. In addition, the humanized antibody may comprise residues not found in either the recipient antibody or the donor antibody. These modifications may be made to further refine antibody performance, such as binding affinity.

In one embodiment, the binding antibody 10 may be an antibody molecule, an immunoglobulin molecule, a fragment thereof, or an aggregate of one or more of them. The binding antibody 10 may have a unique structure that enables specific binding to the target antigen, and each binding antibody 10 may comprise two light chains of identical structure and two heavy chains of identical structure. The heavy chain and the light chain may comprise a variable region and a constant region, respectively. The variable regions of the heavy chain and the light chain may be combined to form an antigen binding site. In the binding antibody 10, a portion to which a target antigen is bound by a protease such as papain may be separated into an antibody binding fragment (Fab) region and a fragment crystallizable (Fc) region, which is a crystallized supporting pillar portion, where the antigen binding site may be included in the antibody binding fragment (Fab) region of the antibody.

Meanwhile, each of the variable regions of the heavy and light chains may have a structure in which three complementarity-determining regions (CDRs) that are hypervariable regions (HVRs), and four framework regions (FRs) structurally supporting the complementarity-determining regions are alternately arranged, respectively. The complementarity-determining regions have different amino acid sequences for each antibody, and thus may be specifically bound to each antigen, whereas the framework regions have conserved amino acid sequences according to subfamilies of organisms, so antibodies of organisms belonging to the same subfamilies have the same or similar amino acid sequences.

For the convenience of description below, in the variable region of the light chain of the binding antibody 10, the four framework regions arranged sequentially apart from the N-terminus, are referred to as first to fourth light chain variable framework regions (VL-FR1, VL-FR2, VL-FR3, and VL-FR4), respectively, and the three complementarity determining regions, which are located between the first to fourth light chain variable framework regions (VL-FR1, VL-FR2, VL-FR3, and VL-FR4), respectively, and sequentially disposed from the N-terminus, are referred to as first to third light chain variable complementarity determining regions (VL-CDR1, VL-CDR2, VL-CDR3), respectively. In addition, in the variable region of the heavy chain of the binding antibody 10, the four framework regions arranged sequentially apart from the N-terminus are referred to as first to fourth heavy chain variable framework regions (VH-FR1, VH-FR2, VH-FR3, and VH-FR4), respectively, and the three complementarity-determining regions, which are located between first to fourth heavy chain variable framework regions (VH-FR1, VH-FR2, VH-FR3, and VH-FR4), respectively, and sequentially disposed from the N-terminus, are referred to as first to third heavy chain variable complementarity-determining regions (VH-CDR1, VH-CDR2, VH-CDR3), respectively.

The peptide compound 110 may have an amino acid sequence capable of selectively and reversibly binding to the fragment antigen-binding (Fab) region of the binding antibody 10.

In one embodiment, the peptide compound 110 may have an amino acid sequence capable of specifically and reversibly binding to one or more of first to fourth framework regions (FR1, FR2, FR3, and FR4) of a light chain or heavy chain of the binding antibody 10. For example, the peptide compound 110 may comprise an amino acid sequence having homology to one or more of the first to fourth framework regions (FR1, FR2, FR3 and FR4) of the light chain or heavy chain of the binding antibody 10. In this case, the peptide compound 110 may be reversibly bound to one or more of the first to fourth framework regions (FR1, FR2, FR3, and FR4) of the light chain or heavy chain. Accordingly, when the target antigen is bound to the binding antibody 10, the peptide compound 10 bound to the binding antibody 10 may be quantitatively released from the binding antibody 10 depending on the amount of the antigen bound to the binding antibody 10.

In one embodiment, the peptide compound 110 may comprise one or more selected from a first peptide compound L1 having a first amino acid sequence having homology to an amino acid sequence of the second light chain variable framework region (VL-FR2), a second peptide compound L2 having a second amino acid sequence having homology to an amino acid sequence of the third or fourth light chain variable framework region (VL-FR3, VL-FR4), a third peptide compound H1 having a third amino acid sequence having homology to an amino acid sequence of the second heavy chain variable framework region (VH-FR2), and a fourth peptide compound H2 having a fourth amino acid sequence having homology to an amino acid sequence of the third or fourth heavy chain variable framework region (VH-FR3, VH-FR4).

In the binding antibody 10, the second light chain variable framework region (VL-FR2) and the third or fourth heavy chain variable framework region (VH-FR3, VH-FR4) are located adjacent to each other and interact with each other, and the third or fourth light chain variable framework region (VL-FR3, VL-FR4) and the second heavy chain variable framework region (VH-FR2) are located adjacent to each other and interact with each other. Thus, the first peptide compound L1 having a first amino acid sequence having homology to the second light chain variable framework region (VL-FR2) of the binding antibody 10 may selectively interact with the third or fourth heavy chain variable framework region (VH-FR3, VH-FR4) of the binding antibody 10, the second peptide compound L2 having a second amino acid sequence having homology to the third or fourth light chain variable framework region (VL-FR3, VL-FR4) of the binding antibody 10 may selectively interact with the second heavy chain variable framework region (VH-FR2) of the binding antibody 10, the third peptide compound H1 having a third amino acid sequence having homology to the second heavy chain variable framework region (VH-FR2) of the binding antibody 10 may selectively interact with the third or fourth light chain variable framework region (VL-FR3, VL-FR4) of the binding antibody 10, and the fourth peptide compound H2 having a fourth amino acid sequence having homology to the third or fourth heavy chain variable framework region (VH-FR3, VH-FR4) of the binding antibody 10 may selectively interact with the second light chain variable framework region (VL-FR2) of the binding antibody 10.

In one embodiment, when the binding antibody 10 is derived from an organism belonging to subfamilies of animals including humans, the peptide compound 110 may comprise about 10 to 25 amino acids and may have a molecular weight of about 1,600 to 3,000 Da. For example, the peptide compound 110 may comprise about 14 to 20 amino acids and may have a molecular weight of about 1,650 to 2,500 Da. In one embodiment, the first to fourth peptide compounds (L1, L2, H1, H2) may comprise amino acid sequences as shown in Table 1 below.

**[Table 1]**

| Switchin 9 peptide | Amino acid sequence | Molecular weight | Gibbs free energy (Kcal/mol) |
|---|---|---|---|
| H1 | SYWIH WVKQR PGQGL EWIGE | 2469.7 | △ G=-17.91 |
| H2 | VYYCA REPTG TGIYF DVWGK | 2325.6 | △ G=-17.19 |
| L1 | TYLEW YPQKP GQSPK LLIYK | 2452.8 | △ G=-15.37 |
| L2 | VYYCF QGSHV PFTK | 1675.9 | △ G=-13. 05 |

Meanwhile, since the framework regions (FR1, FR2, FR3, FR4) of the light and heavy chains of the antibodies have conserved amino acid sequences according to subfamilies of organisms, the peptide compound 110 may be commonly applied to antibodies derived from different species of organisms belonging to the same subfamilies. Therefore, even when the peptide compound 110 is synthesized from antibodies of goat, mouse, rabbit, etc., it can exhibit the same actions and effects as described above for antibodies of human.

The chemical label 120 may be bound to the peptide compound 110 and may be reversibly oxidized and reduced in a detection sample. In one embodiment, the chemical label 120 may comprise one or more selected from the group consisting of ferrocene, ferrocenemethanol, ferrocenedimethanol, α-methylferrocenemethanol, ferrocyanide ion, ferricyanide ion, hexaammineruthenium ion, hydroquinone, ascorbic acid, dopamine, ferrocene carboxylic acid, ferrocene dicarboxylic acid, and ferrocene aldehyde.

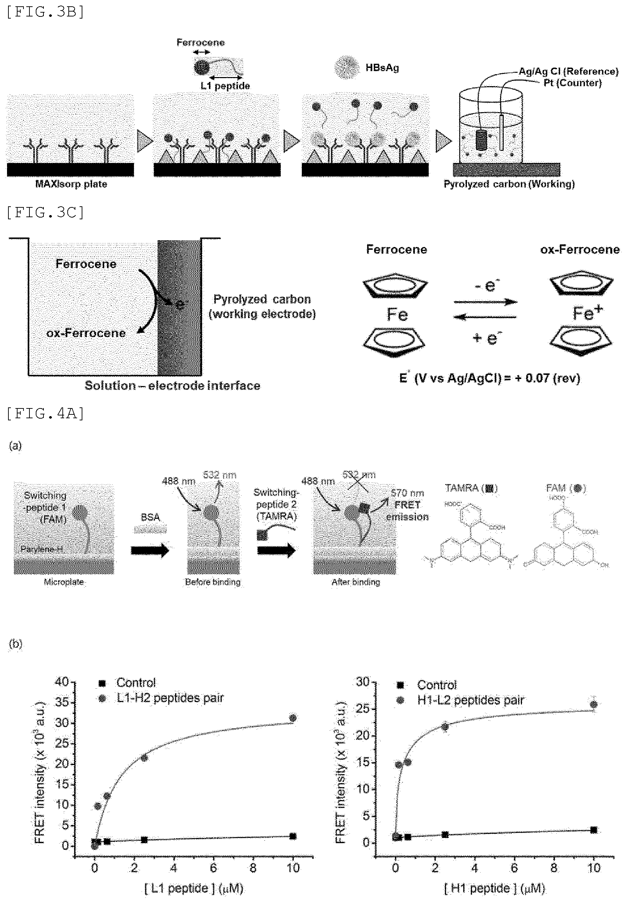

FIG. 3A is a flowchart for explaining an immunoassay method according to an embodiment of the present disclosure, and FIGS. 3B and 3C are schematic diagrams for explaining one embodiment of the immunoassay method illustrated in FIG. 3A.

Referring to FIGS. 3A to 3C, the immunoassay method according to an embodiment of the present disclosure includes: a first step (S110) of immobilizing a binding antibody to which a switching peptide is bound on a substrate or immobilizing the binding antibody on the substrate and then binding the switching peptide to the binding antibody; a second step (S120) of treating the binding antibody with a detection sample solution; and a third step (S130) of quantitatively analyzing a target antigen in the detection sample solution by performing electrochemical analysis on the detection sample solution after the treatment.

In the first step (S110), the switching peptide may comprise a peptide compound having an amino acid sequence capable of selectively and reversibly binding to the fragment antigen-binding (Fab) region of the binding antibody, and a chemical label that is bound to the peptide compound and capable of being oxidized and reduced in the detection sample solution. In one embodiment, as the switching peptide, the switching peptide 100 described with reference to FIGS. 1 and 2 is used. Therefore, an overlapped description thereof will be omitted.

In one embodiment, in the case of the binding antibody and the switching peptide, the binding antibody may be first immobilized on the substrate and then the switching peptide may be bound to the immobilized binding antibody. Alternatively, the switching peptide may be bound to the binding antibody and then the binding antibody may be immobilized on the substrate.

A method of immobilizing the binding antibody on the substrate is not particularly limited. For example, the binding antibody may be directly bound to the substrate, or may be bound to the substrate through a linker compound and immobilized thereto.

As described above, the switching peptide may be selectively and reversibly bound to the fragment antigen-binding (Fab) region of the binding antibody.

In the second step (S120), a detection sample solution may be applied on the bound antibody immobilized on the substrate.

In one embodiment, when there is a target antigen that specifically reacts with the binding antibody in the detection sample solution, if the detection sample solution is applied on the binding antibody, the target antigen may be bound to the binding antibody, and in this case, the switching peptide may be quantitatively released from the binding antibody depending on the amount of the target antigen that has reacted with the binding antibody.

In the third step (S130), the target antigen in the detection sample solution may be quantitatively analyzed by coating the detection sample solution on the binding antibody and performing electrochemical analysis on the detection sample solution after a predetermined period of time has elapsed.

In one embodiment, as described above, when the target antigen reacts with the binding antibody, the peptide compound may be quantitatively released from the binding antibody depending on an amount of the target antigen that has reacted with the binding antibody. In this case, the concentration of the switching peptide in the detection sample solution after the treatment increases, and the electrochemical properties of the detection sample solution may be changed by oxidation or reduction of a chemical label of the switching peptide.

In one embodiment, electrochemical analysis of the detection sample solution after the treatment is not particularly limited. For example, the electrochemical analysis may be performed using methods such as amperometry, chronoamperometry, voltammetry, cyclic voltammetry, differential potential voltammetry, chronopotentiometry, and polarography.

In one embodiment, as illustrated in FIGS. 3A and 3B, when the chemical label of the switching peptide is ferrocene and the electrochemical analysis is performed by a differential potential voltammetry method, the ferrocene of the switching peptide may be oxidized to ferrocene ions by transferring electrons to the working electrode, and thus the electrochemical properties of the detection sample solution may be changed, and as a result, a value of current flowing between a reference electrode and a counter electrode may change. The concentration of the switching peptide contained in the detection sample solution after the treatment changes depending on the amount of the target antigen bound to the binding antibody, and therefore, in the present disclosure, the target antigen may be quantitatively analyzed by measuring the change in the current value. Meanwhile, the ferrocene ion may be reduced to ferrocene by receiving electrons from the working electrode.

According to the switching peptide of the present disclosure and the immunoassay method using the same, by using a switching peptide selectively and reversibly bound to a Fab region of the binding antibody and having a chemical label, the target antigen may be quantitatively analyzed through an electrochemical analysis method that does not require an optical system, enabling miniaturization and low cost of a analysis device, and accordingly, it may be applied to a device for on-site diagnosis.

Hereinafter, specific embodiments of the present disclosure will be described in detail. However, the following examples are merely some embodiments of the present disclosure, and the scope of the present disclosure is not limited to the following examples.

### [Mode for Invention]

### [Example 1]

Four types of peptides (H1, H2, L1, L2) as shown in Table 1 were synthesized. Specifically, the H1 peptide was synthesized to have an amino acid sequence having homology to the FR2 region of the heavy chain, the H2 peptide was synthesized to have an amino acid sequence having homology to the FR3 or FR4 region of the heavy chain, the L1 peptide was synthesized to have an amino acid sequence having homology to the FR2 region of the light chain, and the L2 peptide was synthesized to have an amino acid sequence having homology to the FR3 or FR4 region of the light chain.

A change in Gibbs free energy (ΔG) was -17.91 kcal/mol for the H1 peptide compound, -17.19 kcal/mol for the H2 peptide, 15.37 kcal/mol for the L1 peptide, and -13.05 kcal/mol for the L2 peptide.

### [Experimental Example 1]

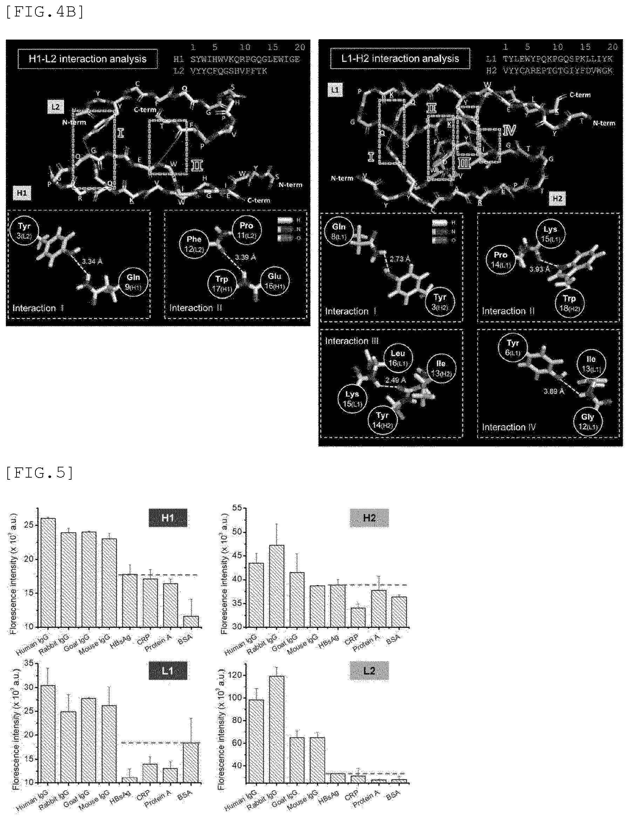

FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software. Here, at the ends of the L1 and L2 peptides, a first fluorescent label, FAM (λₑₓ = 488 nm, λₑₘ = 532 nm) was modified, and at the ends of the H1 and H2 peptides, a second fluorescent label, TAMRA (λₑₓ = 532 nm, λₑₘ = 570 nm) was modified. Meanwhile, the first peptide (L1 or L2) was immobilized on a parylene-H-coated microplate through a covalent bond between a formyl group of parylene-H and a primary amine group of a lysine residue in the peptide, and a second peptide (H2 or H1) was incubated with the immobilized first peptide (L1 or L2) to allow specific interactions between them. On the other hand, it can be seen that a specific interaction between an immobilized first peptide (L1 or L2) and a second peptide (H2 or H1) incubated therewith may be determined by whether the FRET effect with green fluorescence emission from TAMRA (λₑₘ = 570 nm) modified to the second peptide was observed at the bottom of the microplate, and when a specific interaction between the first and second peptides occurs, the FRET effect was observed at the bottom of the microplate.

Referring to FIG. 4A, the interaction between the L1 and H2 peptides was observed by changing the concentration of the L1 peptide, and the FRET intensity increased with increasing the concentration of the L1 peptide. These results indicate that a specific interaction between the L1 and H2 peptides has occurred.

Also, the interaction between the H1 and L2 peptides was observed by changing the concentration of the H1 peptide, and the FRET intensity was observed to increase with increasing the concentration of the H1 peptide. These results also indicate that a specific interaction between the H1 and L2 peptides has occurred.

From the above results, it can be seen that the L1 peptide is selectively and reversibly bound to the FR3 or FR4 region of the heavy chain (corresponding to the H2 peptide), the L2 peptide is selectively and reversibly bound to the FR 2 region of the heavy chain (corresponding to the H1 peptide), the H1 peptide is selectively and reversibly bound to the FR3 or FR4 region of the light chain (corresponding to the L2 peptide), and the H2 peptide is selectively and reversibly bound to the FR2 region of the light chain (corresponding to the L1 peptide).

Referring to FIG. 4B, as a result of analyzing the hydrogen bond between H1-L2 peptides and the hydrogen bond between H2-L1 peptides through PyMOL, in the case of between H1-L2 peptides, (1) a hydrogen bond with a length of 3.34 Å between glutamine (residue number 9 of the H1 peptide) and tyrosine (residue number 3 of the L2 peptide) and (2) a hydrogen bond with a length of 3.39 Å between a carbonyl group of a peptide bond from the L2 peptide and an electron deficient hydrogen of the peptide bond from the H1 peptide were found, and in the case of between H2-L1 peptides, four hydrogen bonds with a length of 2.49 Å to 3.93 Å were found (1) between amino acids (interaction I), (2) between peptide bonds (interactions II, III), and (3) between amino acids and peptide bonds (interaction IV). The number of hydrogen bonds indicates that the interaction between the H1-L2 peptides is stronger than that between the H2-L1 peptides, whereas similar values of change in Gibbs free energy suggest similar binding affinities of peptide compounds to IgG.

FIG. 5 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.

Referring to FIG. 5, only IgGs from human, rabbit, goat, and mouse showed significantly high fluorescence intensities, and other antigenic proteins showed baseline level of fluorescence. These results show that the peptides of Example 1 are bound selectively to the antibody.

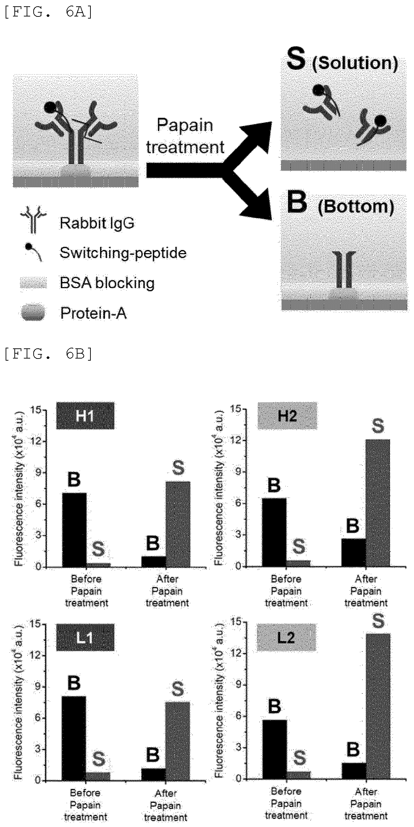

FIG. 6A is a schematic diagram illustrating states before and after treatment of the antibody to which the four types of peptides of Example 1 were bound, respectively, with papain, which is a protease, and FIG. 6B is graphs illustrating a fluorescence intensity measured at a bottom (B) of the microplate and in a solution (S) after the papain treatment. In the case of this experiment, after immobilization of the antibodies (rabbit anti-HRP antibodies) on the microplate, binding between the antibody and the switching peptide was induced by treatment with a fluorescently labeled switching peptide, the unbound switching peptide was removed, and then the Fab region was hydrolyzed from the immobilized antibodies by adding papain, which is a protease.

Referring to FIG. 6A, the fluorescence at the bottom (B) of the microplate is expected to decrease after the papain reaction because the fluorescently labeled switching peptides are bound to the Fab region of antibodies, and the fluorescence in the solution is expected to increase because the Fab region to which the switching peptide is bound is hydrolyzed and dissolved into the solution. This prediction is proven to be true from the graphs of FIG. 6B.

Referring to FIG. 6B, the fluorescence at the bottom (B) of the microplate decreased and the fluorescence in solution (S) increased, after papain treatment compared to before papain treatment in all four types of switching peptides (H1, H2, L1, L2) . Specifically, for the fluorescence intensity at the bottom of the microplate, it was evaluated that the H1 peptide decreased by 84.3%, the H2 peptide decreased by 58.7%, the L1 peptide decreased by 84.8%, and the L2 peptide decreased by 72.5%, respectively. Also, for the fluorescence intensity in the solution, it was evaluated that the H1 peptide increased by 2,184%, the H2 peptide increased by 2,356%, the L1 peptide increased by 1,032%, and the L2 peptide increased by 2,452%, respectively.

From the above results, it can be seen that the four types of switching peptides of Example 1 are bound to the Fab regions of the antibody.

Table 2 below shows the results of evaluating the binding properties of the four types of peptide compounds (H1, H2, L1, L2) of Example 1 to antibodies from different animals (rabbit, mouse, and goat). These results are fluorescence intensity values measured after binding fluorescently labeled switching peptides to antibodies immobilized on a microplate, removing unreacted switching peptides, and then reacting with target antigens (CRP for rabbit IgG, hCG for mouse IgG, HRP for rabbit IgG, HBsAg for goat IgG), respectively.

**[Table 2]**

| Pept ide | Antibody (r=rabbit , g-goat, m-mouse) | In solution (S) | | | Bottom (B) of microplate | | |
|---|---|---|---|---|---|---|---|
| | | Before antigen binding | After antigen binding | Change (%) | Before antigen binding | After antigen binding | Change (%) |
| H1 | Anti-HRP antibody ( r) | 2596(±704) | 21965(±707 8) | +746 | 11840(±29 0) | 1451(±103) | -87.7 |
| H1 | Anti-HBsA 9 | 2242(±91) | 11453(±829 ) | 1410 | 11225(±30 5) | 4618(±71) | -58.9 |
| | antibody( g) | | | | | | |
| H1 | Anti-hCG antibody ( m) | 1281(±63) | 3921(±120) | +206 | 5646(±20) | 1940(±74) | -65.6 |
| H1 | Anti-CRP antibody ( r) | 2242(±91) | 5659(±100) | +152 | 4912(±199 9) | 3595(±30) | -26.8 |
| H2 | Anti-HRP antibody ( r) | 2272(±668) | 18128(±145 6) | +698 | 122230(±3 09) | 1806(±290) | -85.2 |
| H2 | Anti-HBsA g antibody( g) | 1784(±113) | 9136(±835) | +412 | 29367(±76 6) | 18773(±117 6) | -36.1 |
| H2 | Anti-hCG antibody ( m) | 1654(±54) | 8545(±1291 ) | +416 | 23320(±32 60) | 23320(±326 0) | -88.5 |
| H2 | Anti-CRP antibody ( r) | 2099 (H20) | 16311(±165 ) | +677 | 22205 (±73 2) | 22205(±732 ) | -39.5 |
| L1 | Anti-HRP antibody ( r) | 2687(±711) | 12280 (±314 1) | +357 | 15608(±63 8) | 1990 (±251) | -87.3 |
| L1 | Anti-HBsA g antibody( g) | 2779(±293) | 20501(±228 5) | +638 | 11062(±46 26) | 5388 (±581) | -51.3 |
| L1 | Anti-hCG antibody ( m) | 1248 (I76) | 3580 (I416) | +187 | 4630(±676 ) | 1777(±44) | -61.6 |
| L1 | Anti-CRP antibody ( r) | 741 (±197) | 6505(±61) | +778 | 6719(±132 ) | 3456(±178) | -48.6 |
| L2 | Anti-HRP antibody ( r) | 2315(±754) | 16135 (±110 7) | +597 | 12584(±21 0) | 3208 (±117) | -74.5 |
| L2 | Anti-HBsA | 2279 (±635) | 15359 (±405 | +574 | 28002(±31 | 9856(±1150 | -64.8 |
| | g antibody( g) | | 0) | | 6) | ) | |
| L2 | Anti-hCG antibody ( m) | 1692 (±126) | 9402 (±656) | +456 | 33156(±49 80) | 3072 (±184) | -90.7 |
| L2 | Anti-CRP antibody ( r) | 3274 (±353) | 32187(±138 35) | +883 | 20394 (±54 ) | 11236 (I72) | -44.9 |
| L2 | | | | | | | |

Referring to Table 2, it was measured that the fluorescence intensity of each immobilized antibody (IgG) decreased at the bottom of the plate and increased in the solution after treatment with the corresponding antigen. Specifically, in the case of the anti-HRP antibodies from rabbits, for H1, L1, H2, and L2 peptides, the fluorescence intensity at the bottom of the plate decreased by 12.3%, 12.7%, 14.8%, and 25.5%, respectively, and the fluorescence intensity in the solution increased by 746%, 357%, 698%, and 597%, respectively. In the case of anti-HBsAg antibodies from goats, for H1, L1, H2, and L2 peptides, the fluorescence intensity at the bottom of the plate decreased by 41.1%, 48.7%, 63.9%, and 35.2%, respectively, and the fluorescence intensity in the solution increased by 410%, 638%, 412%, and 574%, respectively.

These results show that the four types of peptide compounds synthesized in Example 1 are not only capable of binding to the immobilized antibody (IgG) regardless of the species of the source animal, but are also quantitatively released from the antibody when the antigen was bound to the binding pocket of the antibody due to the reversible binding of each of the four types of peptide compounds of Example 1 to the antibody.

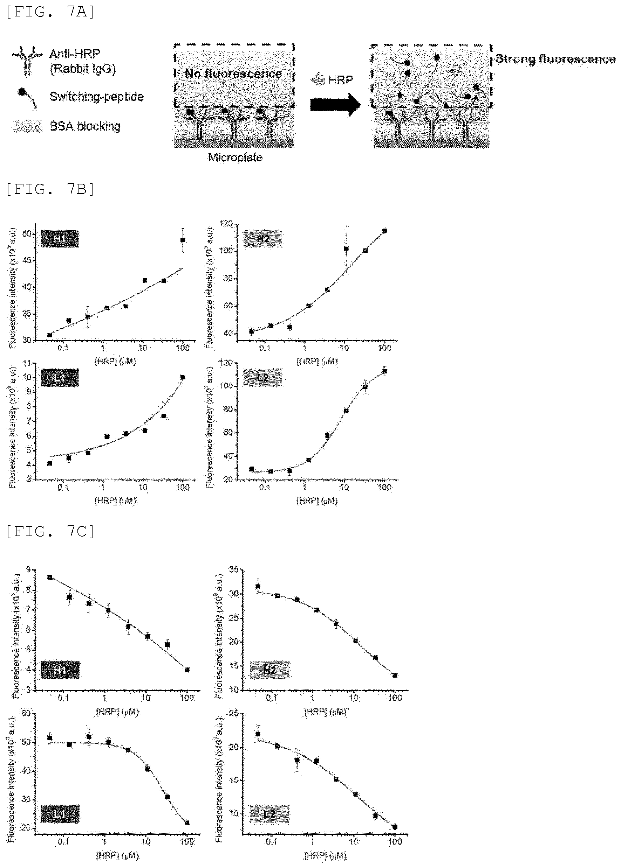

FIG. 7A is a schematic diagram for explaining an immunoassay method using fluorescently labeled peptide compounds of Example 1, and FIGS. 7B and 7C are graphs illustrating the fluorescence intensities in a solution and at a bottom of the microplate depending on a concentration of the antigen measured by the immunoassay method illustrated in FIG. 7A. In this experiment, an anti-HRP antibody from a rabbit immobilized on a microplate was used as the binding antibody and HRP was used as the antigen.

Referring to FIGS. 7A to 7C, as a result of performing immunoassay while varying the concentration of antigen HRP from 0.3 to 100 µM, for the four types of switching peptides (H1, H2, L1, L2), an increase in the fluorescence intensity in the solution was observed depending on the concentration of the antigen (HRP).

Equilibrium dissociation constants of the H1, H2, L1, and L2 peptides were estimated to be 1.65, 3.11, 3.29, and 1.48 µM, and the difference in the dissociation constant for each switching peptide means that each switching peptide has a different detection area for each antigen. For the HRP antigen, the H1 peptide reacted most linearly in the HRP concentration region. These results show that the switching peptide was released from the immobilized antibody according to the bound amount of the antigen, and quantitative analysis of the antigen was possible based on the fluorescence intensity in the solution in one-step immunoassay using the switching peptide.

Meanwhile, FIG. 7C illustrated that the fluorescence intensity at the bottom of the microplate decreased depending on the concentration of the antigen in all four types of switching peptides. These results show that the switching peptide was quantitatively released from the antibody depending on the amount of the antigen, and quantitative analysis of the antigen was possible through a change in the fluorescence intensity in the solution as well as at the bottom of the microplate.

### [Example 2]

Switching peptides were synthesized by binding ferrocene to the terminals of each of the L1, L2, H1, and H2 peptide compounds. Here, the ferrocene was bonded to each end of the peptide compounds through a carboxyl group as shown in Table 3 below.

**[Table 3]**

| Switchin 9 peptide | Amino acid sequence | Molecula r weight (Da) |
|---|---|---|
| L1 | FAM-T-Y-L-E-W-Y-P-Q-K-P-G-Q-S-P-K-L-L-I-Y-K | 2663.34 |
| L2 | FAM-V-Y-Y-C-F-Q-G-S-H-V-P-F-T-K | 1753 |
| H1 | FAM-S-Y-W-I-H-W-V-K-Q-R-P-G-Q-G-L-E-W-I-G-E | 2681.02 |
| H2 | FAM-V-Y-Y-C-A-R-E-P-T-G-T-G-I-Y-F-D-V-W-G-K | 2408.02 |

### [Experimental Example 2]

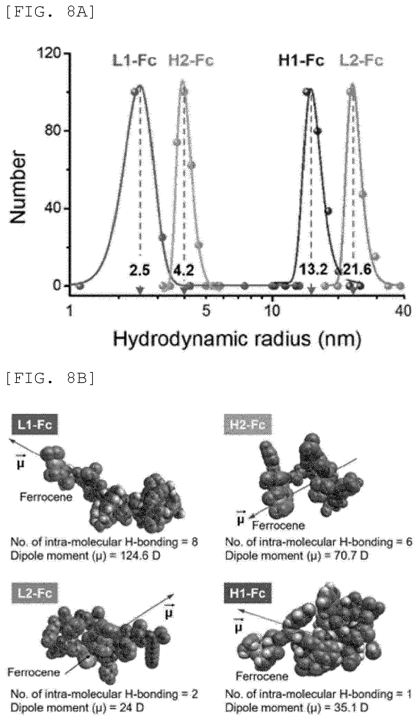

FIGS. 8A to 8C are results of analyzing switching peptides labeled with ferrocene.

FIG. 8A is a result obtained by performing dynamic laser scattering (DLS) to compare the hydrodynamic radius of the switching peptides of the present disclosure. Referring to FIG. 8A, it can be confirmed that DLS was performed using intensity measurement of scattered light under monochromatic laser light, and a hydrodynamic radius of the binding antibody bound to the L1 or H2 peptide was smaller than that of the bound antibody bound to the L2 or H1 peptide. A difference in the hydrodynamic radii of these switching peptides indicates that the contact between the labeled ferrocene and an electrode surface may be sterically hindered depending on the binding relationship between the switching peptides.

Therefore, in order to analyze a difference in a geometry of the four types of switching peptides, three-dimensional structures of the switching peptides were simulated, and the results are shown in FIG. 8B. Referring to FIG. 8B, it can be confirmed that the L1 and H2 peptides have more hydrogen bonds than the L2 and H1 peptides, and a dipole moment direction is closer to that of ferrocene. Therefore, the L1 and H2 peptides may have a compact three-dimensional structure with more intramolecular hydrogen bonds.

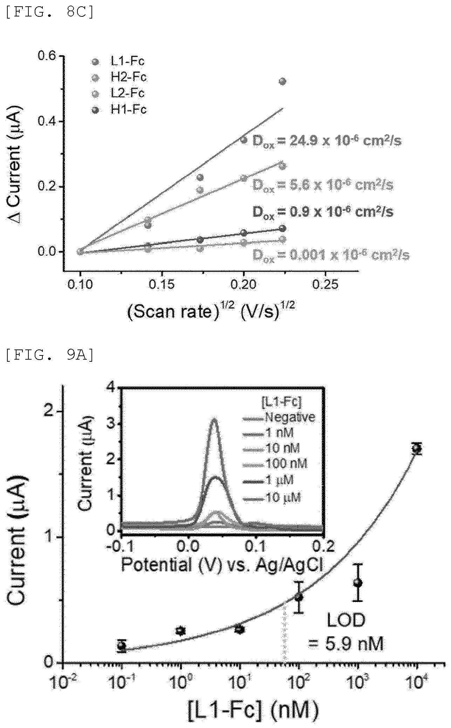

FIG. 8C is a graph obtained by measuring a diffusion coefficient (Dox) of the switching peptides of the present disclosure in CV analysis. Referring to FIG. 8C, the L1 and H2 peptides showed relatively higher diffusion coefficients than the H1 and L2 peptides. Therefore, L1 and H2 peptides labeled with ferrocene may be optimal probes for electrochemical immunoassay.

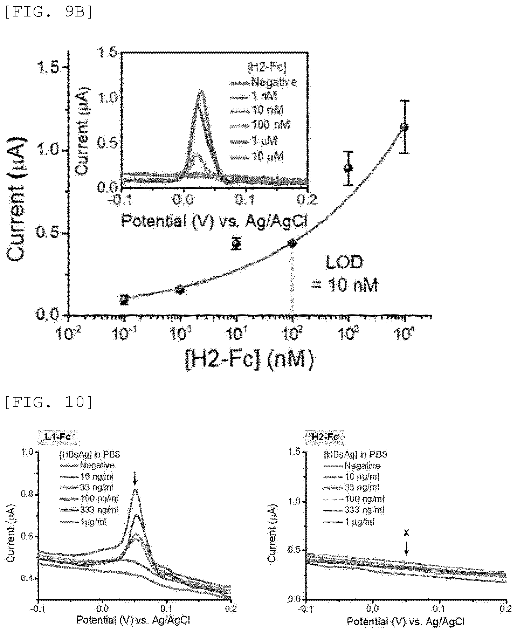

FIGS. 9A and 9B are a graphs illustrating a change in current value depending on the concentration of an L1 switching peptide measured by a differential pulsed voltammetry (DPA) method for a sample solution containing the L1 switching peptide, and a graph illustrating a change in current value depending on the concentration of an H2 switching peptide measured by a differential pulsed voltammetry (DPA) method for a sample solution containing the H2 switching peptide, respectively. Here, a pyrolyzed carbon electrode was used as a working electrode.

Referring to FIG. 9A, the current value measured by a DPA method was found to vary depending on the concentration of the L1 switching peptide. This shows that the antigen may be quantitatively analyzed by a DPA-based immunoassay method using the L1 switching peptide. Referring to FIG. 9B, the current value measured by the DPA method was also found to vary depending on the concentration of an H2 switching peptide. This shows that the antigen may be quantitatively analyzed by the DPA-based immunoassay method using the H2 switching peptide.

In addition, it can be confirmed that the limit of detection (LOD) of the L1 switching peptide was 5.9 nM and the detection limit of the H2 switching peptide was 10 nM. Therefore, the L1 and H2 switching peptides may be used for quantitative analysis of electrochemical immunoassays.

FIG. 10 is the results obtained by performing one-step immunoassay analysis on human hepatitis B antigen (hHBsAg) of the binding antibody bound to the L1 and H2 switching peptides according to the present disclosure.

Referring to FIG. 10, it was confirmed that when the H2 switching peptide was used, the immunoassay for hHBsAg was not possible, and when the L1 switching peptide was used, the immunoassay for hHBsAg was possible. This result is because the L1 switching peptide binds closely near the antigen-binding site, and the binding site of the H2 switching peptide is located relatively far from the antigen-binding site. Therefore, it would be desirable to select the L1 switching peptide for the immunoassay of hHBsAg.

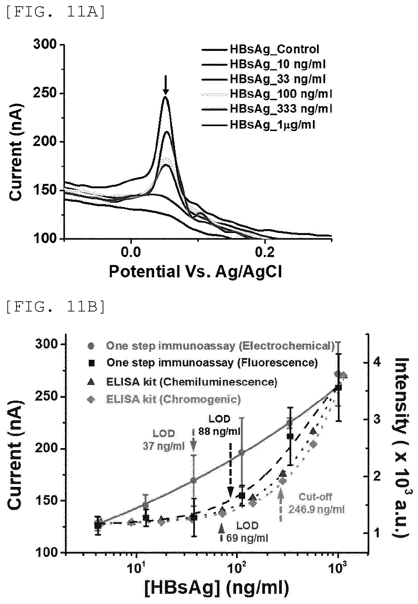

FIGS. 11A and 11B are graphs illustrating analysis results depending on the concentration of the antigen measured through an electrochemical immunoassay method using a first L1 switching peptide (L1 peptide compound having ferrocene bonded to a terminal) for a detection sample solution containing an HBsAg antigen, an optical immunoassay method using a second switching peptide (fluorescently labeled L1 peptide compound), a chemiluminescence-based immunoassay method using a conventional ELISA kit, and a chromogenic immunoassay method using a conventional ELISA kit. The result of the electrochemical immunoassay method using the first L1 switching peptide are measured by the differential pulsed voltammetry (DPA) method for the detection sample solution, after treating the detection sample solution containing the HBsAg antigen with the anti-HBsAg antibody immobilized on the substrate and bound to the L1 switching peptide.

Referring to FIGS. 11A and 11B, it was found that the electrochemical immunoassay method using the first L1 switching peptide was able to measure in a similar range to conventionally commercialized ELISA kit-based immunoassay methods, and in the case of the limit of detection (LOD), the electrochemical immunoassay method using the first L1 switching peptide was more sensitive than other methods.

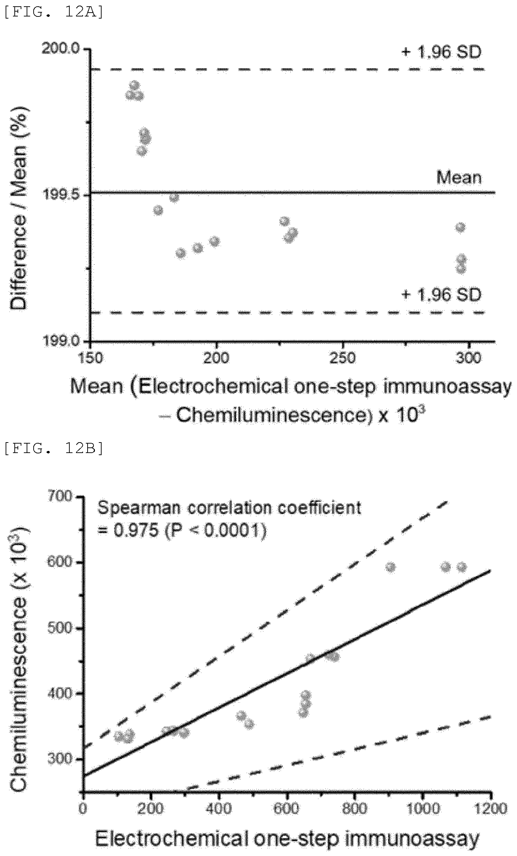

FIGS. 12A and 12B are graphs obtained by performing Bland-Altman test and Passing-Bablok regression analysis for comparison with the conventional method of immunoassay using the peptide compounds of Example 2.

Referring to FIG. 12A, the Bland-Altman test showed that the results of the one-step immunoassay of the present disclosure and ELISA were the same, and were distributed within a confidence range of 95% (±1.96σ). As shown in FIG. 12B, as a result of Passing-Bablok regression analysis, the results of the two methods were distributed within a 95% confidence level, and a Spearman correlation coefficient (ρ) was calculated as 0.975 (P < 0.0001) . Since the Spearman correlation coefficient (ρ) of 0.5 or more shows a high correlation between the two methods, the results of the two statistical analyzes indicate that the results of the ELISA and the immunoassay based on the switching peptide according to the present disclosure are similar. Therefore, it is shown that diagnosis of hepatitis B in humans based on surface antigen (HBsAg) detection is possible using the switching peptide according to the present disclosure.

Although exemplary embodiments of the present disclosure have been disclosed hereinabove, it may be understood by those skilled in the art that the present disclosure may be variously modified and altered without departing from the scope and spirit of the present disclosure described in the following claims.

## Claims

1. A switching peptide comprising:
a peptide compound capable of reversibly binding to a binding antibody; and
a chemical label bound to the peptide compound.

2. The switching peptide of claim 1, wherein the peptide compound is selectively and reversibly bound to a fragment antigen-binding (Fab) region of the binding antibody.

3. The switching peptide of claim 1, wherein the peptide compound has an amino acid sequence capable of specifically and reversibly binding to one or more of first to fourth framework regions (FR1, FR2, FR3, and FR4) of a light chain or heavy chain of the binding antibody.

4. The switching peptide of claim 3, wherein the peptide compound comprises one or more selected from the group consisting of a first peptide compound having a first amino acid sequence having homology to an amino acid sequence of the second light chain variable framework region (VL-FR2) ; a second peptide compound having a second amino acid sequence having homology to an amino acid sequence of the third or fourth light chain variable framework region (VL-FR3, VL-FR4); a third peptide compound having a third amino acid sequence having homology to an amino acid sequence of the second heavy chain variable framework region (VH-FR2) ; and a fourth peptide compound having a fourth amino acid sequence having homology to an amino acid sequence of the third or fourth heavy chain variable framework region (VH-FR3, VH-FR4).

5. The switching peptide of claim 4, wherein the peptide compound comprises 14 to 20 amino acids.

6. The switching peptide of claim 5, wherein the peptide compound has a molecular weight of 1,650 to 2,500 Da.

7. The switching peptide of claim 1, wherein the chemical label comprises a substance capable of being reversibly oxidized or reduced in a sample solution.

8. The switching peptide of claim 7, wherein the chemical label comprises one or more selected from the group consisting of ferrocene, ferrocenemethanol, ferrocenedimethanol, α-methylferrocenemethanol, ferrocyanide ion, ferricyanide ion, hexaammineruthenium ion, hydroquinone, ascorbic acid, dopamine, ferrocene carboxylic acid, ferrocene dicarboxylic acid, and ferrocene aldehyde.

9. An immunoassay method comprising:
a first step of immobilizing a binding antibody to which a switching peptide is bound on a substrate or immobilizing the binding antibody on the substrate and then binding the switching peptide to the binding antibody;
a second step of treating the binding antibody with a detection sample solution; and
a third step of quantitatively analyzing a target antigen that specifically reacts with the binding antibody in the detection sample solution by performing electrochemical analysis on the detection sample solution after the treatment,
wherein the switching peptide comprise a peptide compound having an amino acid sequence capable of selectively and reversibly binding to a fragment antigen-binding (Fab) region of the binding antibody, and a chemical label that is bound to the peptide compound and capable of being oxidized and reduced in the detection sample solution.

10. The immunoassay method of claim 9, wherein when the target antigen is present in the detection sample solution in the second step, if the target antigen is bound to the binding antibody, the switching peptide is quantitatively released from the binding antibody depending on an amount of the target antigen that has reacted with the binding antibody.

11. The immunoassay method of claim 10, wherein the electrochemical analysis is performed by one method selected from the group consisting of amperometry, chronoamperometry, voltammetry, cyclic voltammetry, differential potential voltammetry, chronopotentiometry, and polarography.

12. The immunoassay method of claim 11, wherein a redox reaction occurs between a chemical label of the switching peptide released from the binding antibody in the third step and a working electrode for amperometry or voltammetry.
